# EUROPEAN PATENT APPLICATION

(11) **EP 4 545 044 A1**
(43) Date of publication of application: **30.04.2025**
(21) Application number: 24207518.2
(22) Date of filing: 18.10.2024
(51) Int. Cl.: A61F 2/07

(54) **ADJUSTABLE STENT GRAFT SYSTEMS WITH BRANCH EXTENSIONS**

(30) Priority: 27.10.2023 US 202363546029 P; 15.10.2024 US 202418915939
(71) Applicant: Medtronic Trading NL B.V., 5611ZX Eindhoven (NL); Medtronic Bakken Research Center B.V., 6229 GW Maastricht (NL)
(72) Inventor: ADAMS, Paul, Maastricht (NL); DUIJSENS, Victor Peter Jozef, Maastricht (NL); OVEREEM, Simon Pieter, Maastricht (NL); VAN DER KNAAP, Niels Martinus Josef, Maastricht (NL)
(74) Representative: Zimmermann & Partner Patentanwälte mbB

(57) **Abstract**

An adjustable stent graft system. A first branch extension includes a first main body and a first renal branch. The first main body includes first proximal and distal end regions. The first renal branch is configured to extend into a first renal artery. The first proximal end region is configured to anchor to a connecting platform. The first distal end region is configured to extend into a first common iliac artery (CIA). A second branch extension includes a second main body and a second renal branch. The second main body includes second proximal and distal end regions. The second renal branch is configured to extend into a second renal artery. The second proximal end region is configured to anchor to the connecting platform. The second distal end region is configured to extend into a second CIA. The first and second branch extensions are independently adjustable relative to the connecting platform.

## Description

### CROSS-REFERENCE TO RELATED APPLICATION

This application claims the benefit of U.S. provisional application Serial No. 63/546,029 filed October 27, 2023, and U.S. Patent Application No. 18/915,939, filed October 15, 2024, the disclosures of which are hereby incorporated in its entirety by reference.

### TECHNICAL FIELD

The present disclosure relates to adjustable stent graft systems for endovascular procedures. The adjustable stent graft systems may include first and second branch extensions adjustably connected indirectly to each other through a connecting platform or adjustably connected directly to each other when the first or second branch extension is a bifurcated branch extension.

### BACKGROUND

Endovascular procedures are minimally invasive techniques to deliver clinical treatments in a patient's vasculature (e.g., treatment of aortic aneurysms). One example of a clinical treatment used in an endovascular procedure is deployment of a stent graft. A conventional stent graft typically includes a radially expandable reinforcement structure, e.g., formed from a plurality of annular stent rings, and a cylindrically shaped layer of graft material defining a lumen to which the stent rings are coupled. The stent graft is placed inside a patient's vasculature (e.g., blood vessel) to bridge a diseased blood vessel segment (e.g., an aneurismal, dissected, or torn blood vessel segment), and thereby excluding hemodynamic pressures of blood flow from the diseased blood vessel segment.

### SUMMARY

In an embodiment, a connecting platform for a stent graft system is disclosed. The connecting platform includes a proximal wall having a circumference. The connecting platform further includes a peripheral wall connected to the proximal wall at the circumference. The connecting platform also includes first and second inner walls connected to the proximal wall. The first inner wall is configured to anchor a first branch extension of the stent graft system. The second inner wall is configured to ancho a second branch extension of the stent graft system.

The first and second inner walls may be spaced apart from each other and from the peripheral wall. The peripheral wall includes inner and outer surfaces, and one or more annular sealing rings may extend radially from the outer surface of the peripheral wall. The connecting platform may further include a proximal stent connected to the proximal wall and/or the peripheral wall and extending proximally therefrom. The first inner wall may have a first inner wall diameter, and the second inner wall may have a second inner wall diameter. The first and second inner wall diameters may be coextensive. In one or more embodiments, the proximal wall has a proximal wall diameter coextensive with the first inner wall diameter and the second inner wall diameter. In another embodiment, the proximal wall has a proximal wall diameter spaced apart from the first inner wall diameter and the second inner wall diameter.

In one or more embodiments, the peripheral wall extends in a proximal direction to form a proximal peripheral wall region and extends in a distal direction to form a distal peripheral wall region. The proximal peripheral wall region may define a notch configured to align with a superior mesenteric artery (SMA). The distal peripheral wall region may define one or more notches configured to align with one or more renal arteries.

In another embodiment, an adjustable stent graft system is disclosed. A first branch extension includes a first main body and a first renal branch. The first main body includes first proximal and distal end regions. The first renal branch is configured to extend into a first renal artery. The first proximal end region is configured to anchor to a connecting platform. The first distal end region is configured to extend into a first common iliac artery (CIA). A second branch extension includes a second main body and a second renal branch. The second main body includes second proximal and distal end regions. The second renal branch is configured to extend into a second renal artery. The second proximal end region is configured to anchor to the connecting platform. The second distal end region is configured to extend into a second CIA. The first and second branch extensions are independently adjustable relative to the connecting platform.

In this embodiment, the connecting platform may include first and second inner walls configured to anchor the first and second proximal end regions, respectively. The first and second proximal end regions have first and second proximal end region outer diameters. The first and second inner walls have first and second inner wall diameters. The first and second proximal end region outer diameters are greater than the first and second inner wall diameters, respectively, to anchor the first and second branch extensions to the connecting platform.

The first and second proximal end regions may include outwardly extending fixation features (e.g., proximal stents and/or barbs). In one or more embodiments, the first renal branch has a first nominal renal branch diameter and the second renal branch has a second nominal renal branch diameter different than the first nominal renal branch diameter. In one or more embodiments, the first branch extension has a first branch extension main body length and the second branch extension has a second branch extension main body length different than the first branch extension main body length.

In yet another embodiment, a branch extension of an adjustable stent graft system is disclosed. The branch extension includes a main body including a proximal end region and a distal end region. Th proximal end region is configured to anchor to a connecting platform of the adjustable stent graft system. The distal end region is configured to extend into a common iliac artery (CIA). The branch extension further includes a renal branch extending from the main body and configured to extend to a renal artery. The renal branch has a renal branch distal end flared portion configured to anchor to the renal artery.

The renal branch may be formed of a flexible material configured to transition from a collapsed, delivery position into an extended, deployed position within the renal artery. The distal end region may include a distal end region flared portion configured to anchor to the CIA. The renal branch may have a length of 20 to 25 mm. The renal branch may be configured to independently maintain blood flow between an abdominal aorta and the renal artery without a bridging stent.

### BRIEF DESCRIPTION OF THE DRAWINGS

Figure 1A shows a cross section view of an abdominal aorta and a side view of a stent graft system including a connecting platform and first and second branch extensions connected to the connecting platform.
Figure 1B is an isolated, perspective view of the connecting platform shown in Figure 1A.
Figure 1C is a cut away view of the connecting platform shown in Figure 1A taken along line 1C-1C of Figure 1B.
Figure 1D is an isolated, perspective view of a connecting platform without sealing rings according to another embodiment.
Figure 2A is an isolated, perspective view of a branch extension having a proximal stent according to one embodiment.
Figure 2B is an isolated, perspective view of a first or second branch extension having proximal barbs according to one embodiment.
Figure 2C is an isolated, perspective view of a first or second branch extension having a renal limb in a collapsed position.
Figure 2D is an isolated, perspective view of the first or second branch extension having the renal limb of Figure 2C shown in an extended position.
Figure 2E shows a cross section view of an abdominal aorta and a side view of a stent graft system including a connecting platform and first and second branch extensions connected to the connecting platform where the first and second branch extensions have flared renal limbs and flared distal end regions.
Figure 3A shows a cross section view of an abdominal aorta and a side view of a stent graft system including a connecting platform and first and second branch extensions connected to the connecting platform.
Figure 3B is an isolated, top perspective view of the connecting platform shown in Figure 3A.
Figure 3C is an isolated, side perspective view of the connecting platform shown in Figure 3A.
Figure 3D is a cut away view of the connecting platform shown in Figure 3A taken along line 3D-3D of Figure 3C.
Figure 3E is a schematic, top view of the connecting platform shown in Figure 3A showing the relative orientation of a superior mesenteric artery (SMA) notch and proximal ends of first and second branch extensions.
Figure 4A shows a cross section view of an abdominal aorta and a side view of a stent graft system including a bifurcated connecting platform and first and second branch extensions connected to the connecting platform.
Figure 4B is an isolated, cut away view of the bifurcated connecting platform shown in Figure 4A taken along line 4B-4B of Figure 4A.
Figure 4C is an isolated, cut away view of the first branch extension shown in Figure 4A taken along line 4C-4C of Figure 4A.
Figure 4D is an isolated, cut away view of the second branch extension shown in Figure 4A taken along line 4D-4D of Figure 4A.
Figure 5A shows a cross section view of an abdominal aorta and a side view of a stent graft system including a bifurcated first branch extension and a second branch extension connected to the bifurcated first branch extension.
Figure 5B is an isolated, fragmented, cut away view of the bifurcated first branch extension shown in Figure 5A taken along line 5B-5B of Figure 5A.
Figure 5C is an isolated, fragmented, cut away view of the second branch extension shown in Figure 5A taken along 5C-5C of Figure 5A.
Figure 6A is a schematic, side view of a connecting platform according to an embodiment.
Figure 6B is a schematic, top view of the connecting platform according to the embodiment shown in Figure 6A.
Figure 6C is a cross section view of the connecting platform shown in Figure 6A where first and second branch extensions are attached thereto.
Figure 7A is a schematic, side view of a connecting platform including a foam insert according to an embodiment.
Figure 7B is a schematic, top view of the foam insert of the connecting platform shown in Figure 7A.
Figure 7C is a cross section view of the connecting platform shown in Figure 7A in a deployed position where first and second branch extensions have formed first and second apertures, respectively, in the foam insert.
Figure 7D is a schematic, top view of the foam insert in the deployed position showing the first and second apertures in the foam insert.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical applications. Various combinations and modifications of the features consistent with the teachings of this disclosure, however, could be desired for particular applications or implementations.

Directional terms used herein are made with reference to the views and orientations shown in the exemplary figures. A central axis is shown in the figures and described below. Terms such as "outer" and "inner" are relative to the central axis. For example, an "outer" surface means that the surfaces faces away from the central axis, or is outboard of another "inner" surface. Terms such as "radial," "diameter," "circumference," etc. also are relative to the central axis. The terms "front," "rear," "upper" and "lower" designate directions in the drawings to which reference is made.

Unless otherwise indicated, for the delivery system the terms "distal" and "proximal" are used in the following description with respect to a position or direction relative to a treating clinician. "Distal" and "distally" are positions distant from or in a direction away from the clinician, and "proximal" and "proximally" are positions near or in a direction toward the clinician. For the stent-graft prosthesis, "proximal" is the portion nearer the heart by way of blood flow path while "distal" is the portion of the stent-graft further from the heart by way of blood flow path.

The following detailed description is merely exemplary in nature and is not intended to limit the invention or the application and uses of the invention. Although the description is in the context of treatment of blood vessels such as the aorta, coronary, carotid, and renal arteries, the invention may also be used in any other body passageways (e.g., aortic valves, heart ventricles, and heart walls) where it is deemed useful.

A stent graft may be deployed to bridge an aortic aneurism using endovascular aneurysm repair (EVAR). However, some patients are no eligible for an EVAR procedure when the aneurysm is too close to one or more side branches (e.g., the left renal artery, the second renal artery, and/or superior mesenteric artery (SMA)) of the aorta. The stent graft may block one or more of those branches, thereby stopping blood flow to one or more organs.

Proposed solutions include fenestrated and/or branched endovascular aortic repair (F-BEVAR). F-BEVAR may use a custom-made device based on a patient's computed tomography (CT) scans, thereby providing a device with side branches and/or fenestrations aligning with the branch arteries, thereby maintaining blood flow to the patient's organs. However, the F-BEVAR procedure may suffer from one or more drawbacks (e.g., the custom-made device may take up to six (6) weeks to manufacture, the custom-made device may be several times more expensive than a standard EVAR device, and/or the F-BEVAR device has limited applicability).

What is desired is a stent graft system that addresses one or more of the drawbacks of a custom-made device and/or an F-BEVAR device while providing a solution for patients with challenging anatomy. One or more of the embodiments disclosed herein provide an adjustable stent graft system with branch extensions. The components and sizes of the adjustable stent graft system may be standard across patients while providing adjustability relative to each of the standard components.

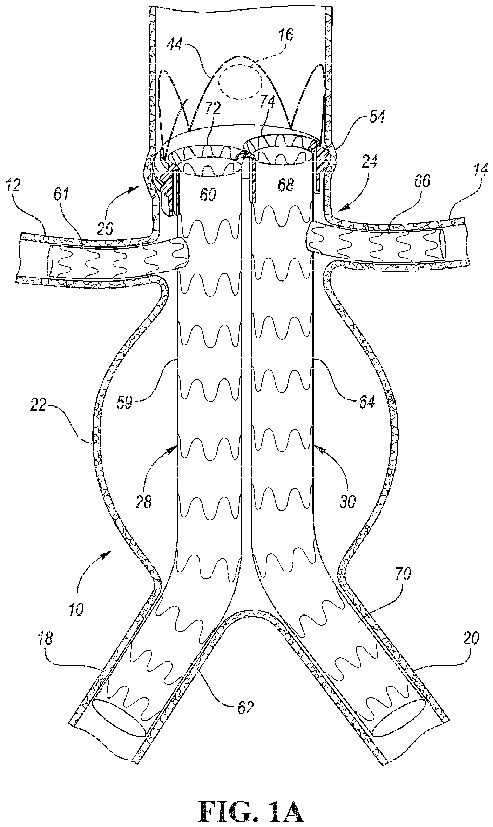

Figure 1A depicts a cross section view of abdominal aorta 10 and right renal artery 12 and left renal artery 14 extending from abdominal aorta 10. Right renal artery 12 and left renal artery 14 may be referred to generally as the renal arteries. Superior mesenteric artery (SMA) 16 branches from abdominal aorta 10. Abdominal aorta 10 branches into first common iliac artery (CIA) 18 and second CIA 20. Abdominal aorta 10 includes aneurysm 22. Figure 1A depicts a side view of stent graft system 24 bridging aneurysm 22 and includes connecting platform 26 with first branch extension 28 and second branch extension 30 connected thereto.

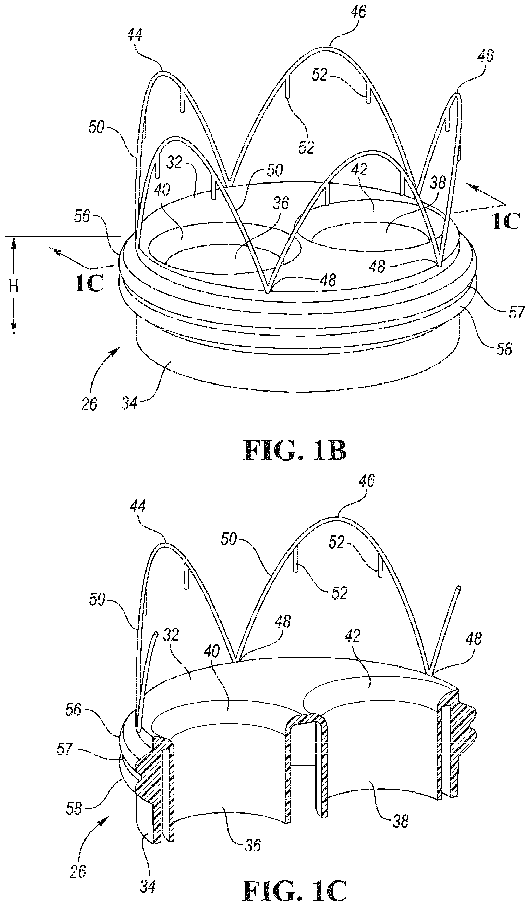

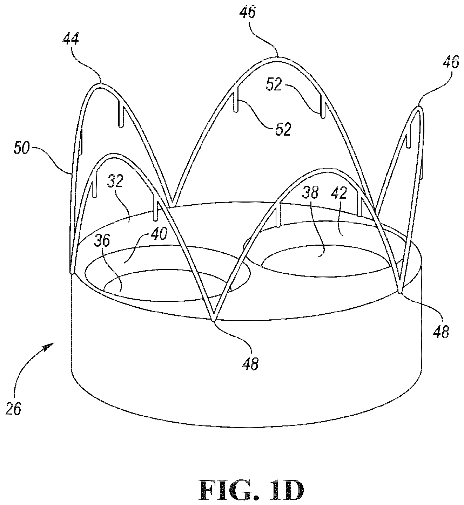

As shown in Figures 1B, 1C, and 1D, connecting platform 26 includes proximal wall 32 extending into peripheral wall 34 and first inner wall 36 and second inner wall 38. As shown in Figures 1B, 1C, and 1D, proximal wall 32 has a circular profile and peripheral wall 34 has a cylindrical profile, and first inner wall 36 and second inner wall 38 have cylindrical profiles. As best shown in Figure 1C, first inner wall 36 and second inner wall 38 may be spaced apart from peripheral wall 34 and each other. First inner wall 36 and second inner wall 38 have first and second longitudinal axes, respectively, which may be parallel to each other such that first inner wall 36 and second inner wall 38 are spaced apart from each other along their height. The first and second longitudinal axes of first inner wall 36 and second inner wall 38 may be orthogonal to proximal wall 32. As shown in Figures 1B, 1C, and 1D, diameters of first inner wall 36 and second inner wall 38 are coextensive with each other and coextensive with a diameter of proximal wall 32. First inner wall 36 and second inner wall 38 may run the height of connecting platform 26. In other embodiments, first inner wall 36 and/or second inner wall 38 may be shorter than the height of connecting platform 26.

Proximal wall 32 transitions into first inner wall 36 and second inner wall 38 through first chamfered portion 40 and second chamfered portion 42, respectively. First chamfered portion 40 and second chamfered portion 42 provide a smooth transition between proximal wall 32 and first inner wall 36 and second inner wall 38 to avoid sharp edges within these transition regions. As best shown in Figure 1C, first chamfered portion 40 and second chamfered portion 42 are convex relative to the first and second longitudinal axes, respectively, of first inner wall 36 and second inner wall 38, respectively. In other embodiments, first chamfered portion 40 and/or second chamfered portion 42 may be concave.

Connecting platform 26 includes proximal stent 44 extending proximally beyond connecting platform 26. Proximal stent 44 includes proximal crowns 46, distal crowns 48, and struts 50 extending between proximal crowns 46 and distal crowns 48 in an alternative repeating arrangement of a sinusoidal ring. Distal crowns 48 are secured to proximal wall 32 of connecting platform 26. Distal crowns 48 may be molded to proximal wall 32 when connecting platform 26 is formed of a polymeric material. Distal crowns 48 may be stitched or sutured to proximal wall 32 of connecting platform 26. Proximal stent 44 is configured to extend beyond the circumference of peripheral wall 32 to anchor to an inner wall of abdominal aorta 10. As shown in Figure 1A, proximal stent 44 is anchored to inner wall of abdominal aorta 10 such that it does not interfere with blood flow through SMA 16. Proximal stent 44 may further include barbs 52 extending distally from struts 50 adjacent to proximal crowns 46. Barbs 52 may be formed at locations on both sides of a proximal stent 44 equidistant from the proximal stent 44. Barbs 52 may be configured to enhance the anchoring of proximal stent 44 to the inner vessel wall.

Connecting platform 26 may be deployed using a guidewire introduced by femoral access. The guidewire may be inserted into the femoral artery and routed up through abdominal aorta 10. A delivery system including connecting platform 26 in a compressed, delivery state may be introduced via femoral access. The delivery system may be advanced into abdominal aorta 10 over the guidewire. The delivery system may be positioned at a desired location (e.g., landing zone 54 between right renal artery 12 and left renal artery 14 and SMA 16) such that connecting platform 26 is positioned at the desired location. A delivery sheath of the delivery system may be withdrawn to release connecting platform 26 into an expanded, deployed position at landing zone 54. In the expanded, deployed position, connecting platform 26 exerts and outward radial force on the inner vessel wall to anchor and to seal connecting platform 26 to landing zone 54 to resist blood flow between connecting platform 26 and the inner vessel wall.

As shown in Figures 1A through 1C, first sealing region 56 and second sealing region 58 (e.g., first and second sealing annular rings) extend radially from peripheral wall 34. First sealing region 56 and second sealing region 58 may be spaced apart from each other to from valley 57 therebetween. First sealing region 56 and second sealing region 58 may extend the entire circumference of peripheral wall 34 to enhance the sealing between the inner vessel wall and connecting platform 26. First sealing region 56 and second sealing region 58 may be capped with a polymeric material having a higher coefficient of friction than the peripheral wall 34 to enhance the anchoring of connecting platform 26 to the inner vessel wall. The polymeric material may include hooks or other surface features to enhance the grip of the peripheral wall of the connecting platform to the inner wall of the vessel. In another embodiment shown in Figure 1D, connecting platform 26 does not include any separate sealing regions or annular rings.

In one or more embodiments, the height H of connecting platform 26 may be any of the following values or in a range of any two of the following values: 10.0, 10.5, 11.0, 11.5, and 12.0 mm. The average distance between the highest renal artery and the SMA may be about 18.0 mm. Accordingly, in some embodiments, the height of connecting platform 26 may be longer provided that connecting platform 26 does not overlap with or partially or completely block the SMA and one or more renal arteries. The overall diameter of connecting platform 26 may be any of the following values or in a range of any two of the following values: 23, 24, 25, 26, 27, 28, 29, 30, 31, 32, 33, 34, 35, and 36 mm. The overall diameter of connecting platform 26 may be selected such that connecting platform 26 anchors to and seals with the inner vessel wall at the landing zone between the renal arteries and the SMA. Connecting platform 26 may be formed of a flexible material (e.g., a polymeric mesh material) configured to permit connecting platform 26 to conform to the inner vessel wall diameter while maintaining a seal. In one or more embodiments, a proximal side region (e.g., a portion of peripheral wall 34) may include a number of fixation hooks to enhance the fixation of connecting platform 26 to the inner vessel wall at the landing area.

Stent graft system 24 also includes first branch extension 28 and second branch extension 30 connected to connecting platform 26. First branch extension 28 and second branch extension 30 may be expandable and configured for delivery via femoral access. First and second branch extensions 28 and 30 may be congruent or similar in shape to each other. First branch extension 28 may be implanted into connecting platform 26 separately from second branch extension 30 such that the height of first branch extension 28 and second branch extension 30 may vary relative to connecting platform 26.

First branch extension 28 includes first main body 59 and first renal branch 61 extending from first main body 59. First renal branch 61 is configured to extend into right renal artery 12 to permit perfusion of blood from abdominal aorta 10 into right renal artery 12. First branch extension 28 includes first proximal end region 60 and first distal end region 62. First proximal end region 60 may be configured to anchor to first inner wall 36 of connecting platform 26. First distal end region 62 may be configured to extend into first CIA 18 to permit perfusion of blood from abdominal aorta 10 into first CIA 18. Second branch extension 30 includes second main body 64 and second renal branch 66 extending from second main body 64. Second renal branch 66 is configured to extend into left renal artery 14 to permit perfusion of blood from abdominal aorta 10 into left renal artery 14. Second branch extension 30 includes second proximal end region 68 and second distal end region 70. Second proximal end region 68 may be configured to anchor to second inner wall 38 of connecting platform 26. Second distal end region 70 may be configured to extend into second CIA 20 to permit perfusion of blood from abdominal aorta 10 into second CIA 20. First proximal end region 60 and/or second proximal end region 68 may have an irregular cross section (e.g., a D-shaped cross section) to enhance sealing within first inner wall 36 and second inner wall 38, respectively. In other embodiments, proximal regions 60 and 68 may have circular cross sections.

The outer diameter of a renal branch may be any of the following values or in a range of any two of the following values: 5.0, 5.5, 6.0, 6.5, 7.0, 7.5, and 8.0 mm. The length of a renal branch may be any of the following values or in a range of any two of the following values: 20.0, 20.5, 21.0, 21.5, 22.0, 22.5, 23.0, 23.5, 24.0, 24.5, and 25.0 mm. The renal branch may be fixed or collapsible depending on the embodiment.

First branch extension 28 and second branch extension 30 may be formed of a graft material. The graft material may be any blood impermeable material (e.g., low-porosity woven or knit polyester, DACRON^{®} material, expanded polytetrafluoroethylene, polyurethane, and silicone). The graft material may be a natural material (e.g., a membranous tissue material such as pericardium or intestinal submucosa). First branch extension 28 and second branch extension 30 may include circumferential stents coupled to the graft material. The circumferential stents may be self-expanding members formed from a shape memory material (e.g., a nickel-titanium alloy such as nitinol). In another embodiment, the circumferential stents may be balloon expandable stents.

The inner diameters of first inner wall 36 and second inner wall 38 and the outer diameters of first proximal end region 60 and second proximal end region 68 are selected such that first branch extension 28 and second branch extension 30 adequately fixate to connecting platform 26. In one or more embodiments, first proximal end region 60 has a greater outer diameter than an inner diameter of first inner wall 36, and second proximal end region 68 has a greater outer diameter than an inner diameter of second inner wall 38. The outer diameters of first proximal end region 60 and/or second proximal end region 68 may be any of the following values or in a range of any two of the following values: 15.5, 15.6, 15.7, 15.8, 15.9, 16.0, 16.1, 16.2, 16.3, 16.4, and 16.5 mm. The inner diameters of first inner wall 36 and/or second inner wall 38 may be any of the following values or in a range of any two of the following values: 13.5, 13.6, 13.7, 13.8, 13.9, 14.0, 14.1, 14.2, 14.3, 14.4, and 14.5 mm. The outer diameters of the proximal end regions being greater than the inner diameters of the connecting platform walls provide radial pressure to enhance fixation between the branch extensions and the connecting platform along the height of the connecting platform. In one or more embodiments, the radial pressure overcomes a relatively short engagement along the height of the inner walls to resist dislodgement of the branch extensions from the inner walls.

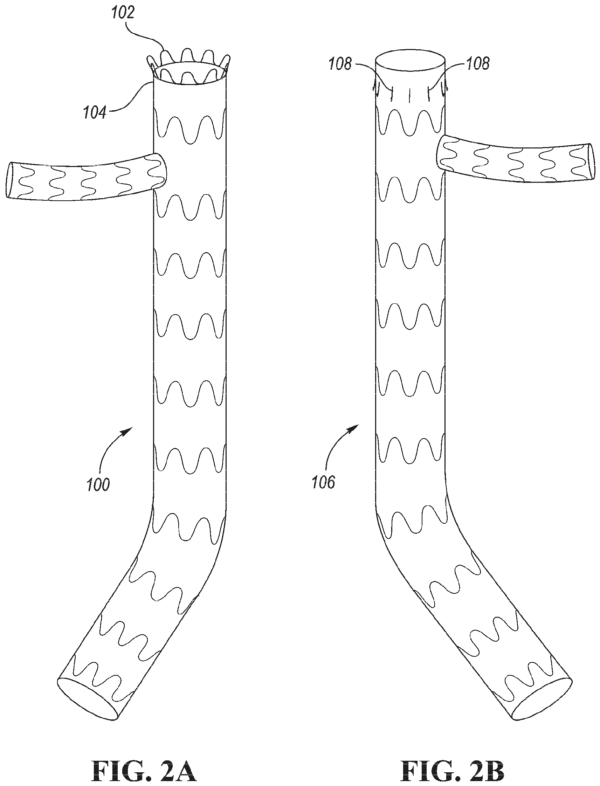

Figure 2A depicts branch extension 100 having proximal stent 102 configured to enhance a clamping force within an inner wall or a chamfered portion of a connecting platform (e.g., first inner wall 36 and/or second inner wall 38 or first chamfered portion 40 and/or second chamfered portion 42). As shown in Figure 2A, proximal stent 102 extends from proximal end 104 of branch extension 100. Proximal stent 102 may have a sinusoidal shape that runs the circumference of proximal end 104. Figure 1A depicts first proximal stent 72 and second proximal stent 74 lodged against first chamfered portion 40 and second chamfered portion 42 to further anchor first branch extension 28 and second branch extension 30, respectively.

Figure 2B depicts branch extension 106 having spaced apart barbs 108 configured to enhance a clamping force within an inner wall or a chamfered portion of a connecting platform (e.g., first inner wall 36 and/or second inner wall 38 or first chamfered portion 40 and/or second chamfered portion 42). As shown in Figure 2B, spaced apart barbs 108 are anchored around a circumference of extension branch 106 and extending downwardly and outwardly from the anchor points. Spaced apart barbs 108 may extend around the entire circumference of branch extension 106. The number of spaced apart barbs around a circumference of an extension branch may be any of the following values or in a range of any two of the following values: 8, 9, 10, 11, 12, 13, and 14. The proximal stents and barbs may be referred to as examples of outwardly extending fixation features.

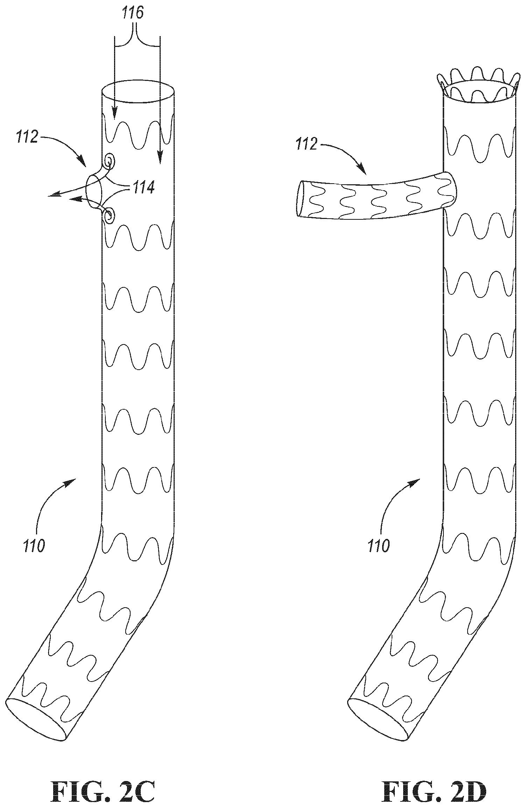

Figure 2C is an isolated, perspective view of branch extension 110 having renal limb 112 in a collapsed position. Renal limb 112 may be formed of a flexible material (e.g., a medical balloon-type material). The medical balloon-type material may be polyurethane, Pebax^{®}, nylon, polyester, polyethylene terephthalate (PET), or a combination thereof. Figure 2D is an isolated, perspective view of branch extension 110 with renal limb 112 in an extended position. Renal limb 112 expands from the collapsed position to the extended position into a renal artery as shown by arrows 114 by blood pressure caused by blood flowing through the abdominal aorta and branch extension 110 as shown by arrows 116. In its expanded position shown in Figure 2D, renal limb 112 extends within a renal artery. Renal limb 112 may also be extended into a renal artery by wire release or balloon capture and extension. In one or more embodiments, the renal limb may extend an adequate length within a renal artery such that a bridging stent graft is not used to maintain blood flow between the abdominal artery and the renal artery. The renal limb may have a conical, tapered shape where it aligns with an entry to the renal artery to enhance sealing of the renal limb to the renal artery. In other embodiments, the renal limb 112 may be formed as a branch, coupling, port, or fenestration that does not extend into the renal artery or extends only a slight distance therein. In these embodiments, a bridging stent graft is deployed within the renal limb 112 to extend into and seal with the renal artery. The bridging stent graft may be self-expanding or balloon-expanding.

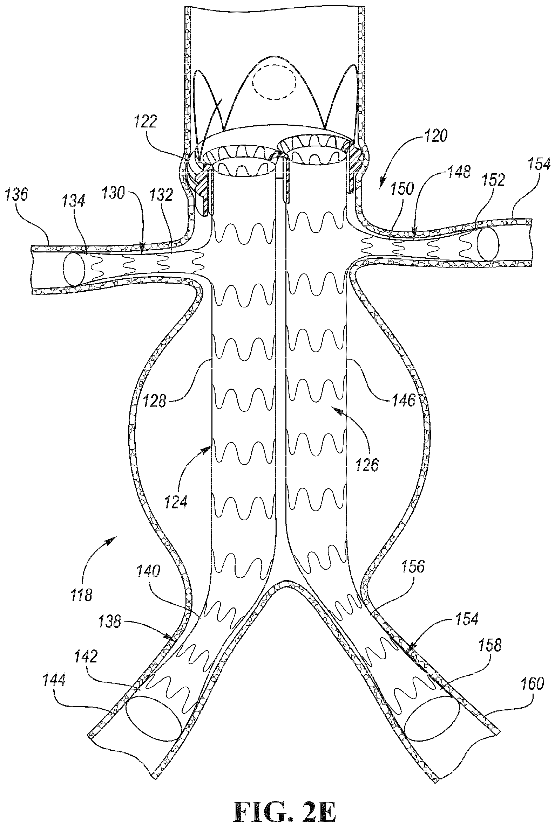

Figure 2E shows a cross section view of abdominal aorta 118 and a side view of stent graft system 120 including connecting platform 122 and first branch extension 124 and second branch extension 126 connected to connecting platform 122.

First branch extension 124 includes first main body 128 and first renal branch 130 extending from first main body 128. First renal branch 130 includes first renal branch proximal end region 132 and first renal branch distal end region 134. First renal branch proximal end region 132 flares into first renal branch distal end region 134 such that first renal branch proximal end region 132 has a smaller nominal diameter than first renal branch distal end region 134, thereby creating a clearance at the entry into right renal artery 136 from abdominal aorta 118 and anchoring first renal branch 130 at flared distal end region 134 to the inner wall of right renal artery 136. First main body 128 includes first distal end region 138 having first proximal portion 140 and first distal portion 142. First proximal portion 140 flares into first distal portion 142 such that first proximal portion 140 has a smaller nominal diameter than first distal portion 142, thereby creating a clearance at the entry into first CIA 144 from abdominal aorta 118 and anchoring first branch extension 124 at flared distal portion 142 to the inner wall of first CIA 144.

Second branch extension 126 includes second main body 146 and second renal branch 148 extending from second main body 146. Second renal branch 148 includes second renal branch proximal end region 150 and second renal branch distal end region 152. Second renal branch proximal end region 150 flares into second renal branch distal end region 152 such that second renal branch proximal end region 150 has a smaller nominal diameter than second renal branch distal end region 152, thereby creating a clearance at the entry into left renal artery 154 from abdominal aorta 118 and anchoring second renal branch 148 at flared distal end region 152 to the inner wall of left renal artery 154. Second main body 146 includes second renal branch 148 having second proximal portion 156 and second distal portion 158. Second proximal portion 156 flares into second distal portion 158 such that second proximal portion 156 has a smaller nominal diameter than second distal portion 158, thereby creating a clearance at the entry into second CIA 160 from abdominal aorta 118 and anchoring second branch extension 126 at flared distal portion 158 to the inner wall of second CIA 160.

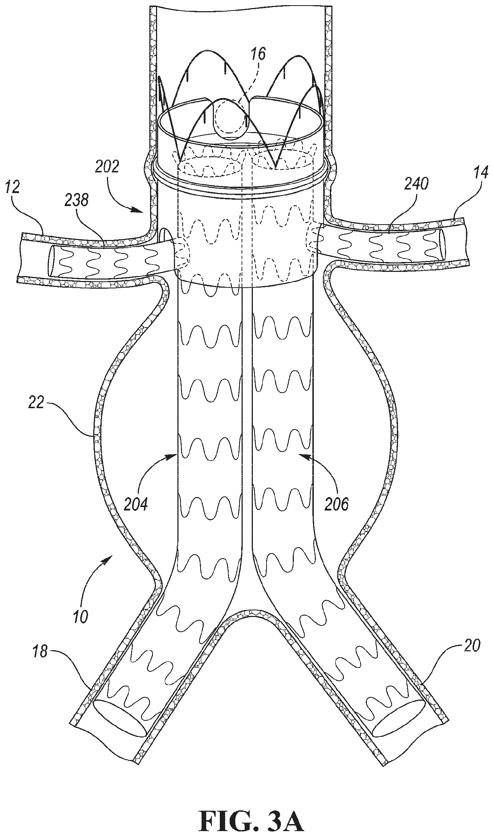

Figure 3A depicts a side view of stent graft system 200 bridging aneurysm 22 within abdominal aorta 10. Right renal artery 12 and left renal artery 14 extends from abdominal aorta 10. Superior mesenteric artery (SMA) 16 branches from abdominal aorta 10. Abdominal aorta 10 branches into first common iliac artery (CIA) 18 and second CIA 20. Stent graft system 200 includes connecting platform 202 with first branch extension 204 and second branch extension 206 connected thereto.

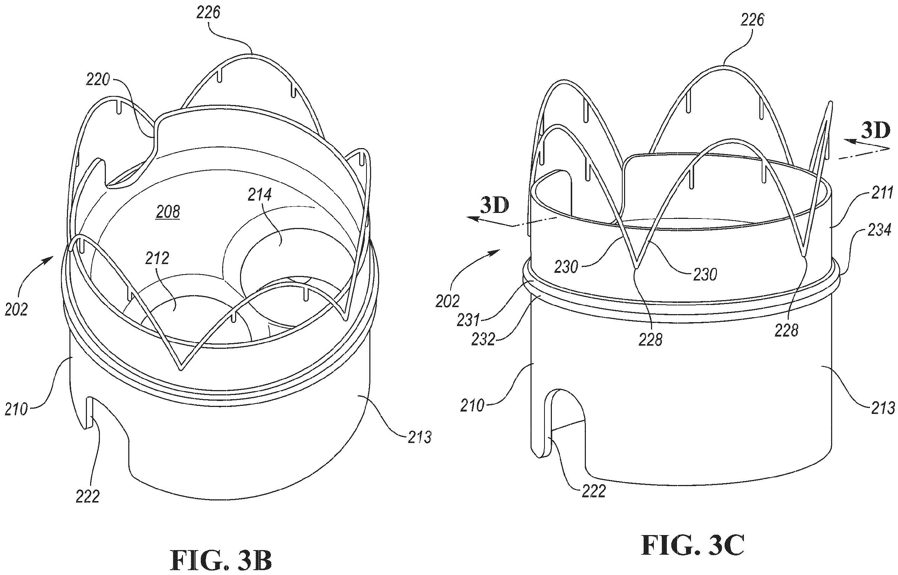

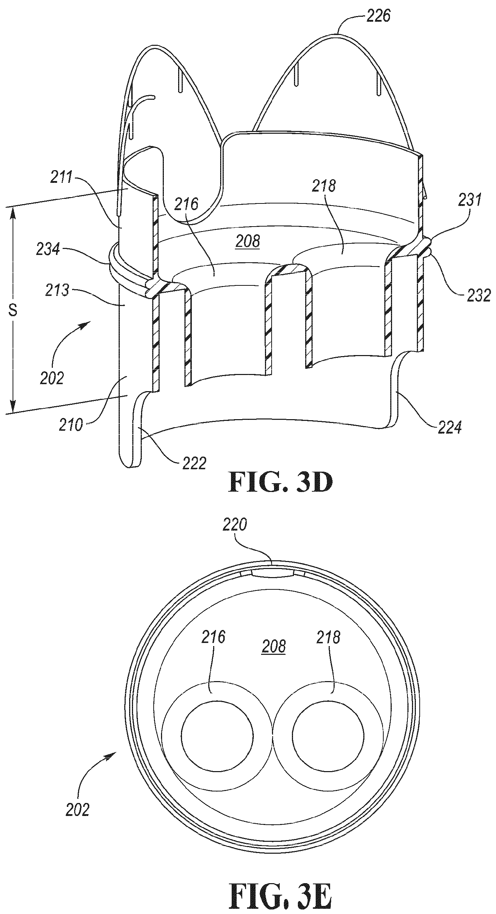

As shown in Figures 3A, 3B, 3C, 3D, and/or 3E, connecting platform 202 includes proximal wall 208 extending into peripheral wall 210 and first inner wall 212 and second inner wall 214. Proximal wall 208 has a circular profile and peripheral wall 210 has a cylindrical profile, and first inner wall 212 and second inner wall 214 have cylindrical profiles. As best shown in Figure 3D, first inner wall 212 and second inner wall 214 may be spaced apart from peripheral wall 210 and each other. First inner wall 212 and second inner wall 214 have first and second longitudinal axes, respectively, which may be parallel to each other such that first inner wall 212 and second inner wall 214 are spaced apart from each other along their height. As shown in Figures 1B, 1C, and 1D, diameters of first inner wall 212 and second inner wall 214 are coextensive with each other and are spaced apart from a parallel diameter of proximal wall 208 such that first inner wall 212 and second inner wall 214 are spaced toward one side of proximal wall 208. Peripheral wall 210 may extend in first and second directions (e.g., first and second directions parallel to a longitudinal axis of peripheral wall 210) away from proximal wall 208. As shown in Figure 3D, peripheral wall 210 extends in the first direction (e.g., proximal direction) to form proximal peripheral wall region 211 a shorter height than in the second direction (e.g., distal direction) to form distal peripheral wall region 213. First inner wall 212 and second inner wall 214 may run from proximal wall 208 in the second, distal direction. First inner wall 212 and/or second inner wall 214 may be shorter than the height of connecting platform 202.

Proximal wall 208 transitions into first inner wall 212 and second inner wall 214 through first chamfered portion 216 and second chamfered portion 218, respectively. First chamfered portion 216 and second chamfered portion 218 provide a smooth transition between proximal wall 208 and first inner wall 212 and second inner wall 214 to avoid sharp edges within these transition regions. As best shown in Figure 3D, first chamfered portion 216 and second chamfered portion 218 are convex relative to the first and second longitudinal axes, respectively, of first inner wall 212 and second inner wall 214, respectively. In other embodiments, first chamfered portion 216 and/or second chamfered portion 218may be concave.

As discussed herein, peripheral wall 210 includes proximal peripheral wall region 211 and distal peripheral wall region 213. Peripheral wall region 211 defines first notch 220, which may have flat sides and a curved bottom (e.g., a scallop-shaped notch), at an edge thereof. In one or more embodiments, first notch 220 is sized to align with SMA 16 when connecting platform 202 is in an expanded, deployed position so that connecting platform 202 does not act as an impediment to blood flow between SMA 16 and abdominal aorta 10. Distal peripheral wall region 213 defines second notch 222 and third notch 224 opposing each other at an edge of distal peripheral wall region 213. Second notch 222 and third notch 224 may each have flat sides and a curved bottom (e.g., scallop-shaped notches). In one or more embodiments, second notch 222 and third notch 224 are sized to align with right renal artery 12 and left renal artery 14, respectively, when connecting platform 202 is in an expanded, deployed position so that connecting platform 202 does not act as an impediment to blood flow between right renal artery 12 and left renal artery 14 and abdominal aorta 10. Second notch 222 and third notch 224 may also be configured to provide renal limb 238 and renal limb 240 of first branch extension 204 and second branch extension 206, respectively, access to the renal arteries.

In one embodiment, first notch 220 is at a twelve o'clock orientation whereas second notch 222 and third notch 224 are at three o'clock and nine o'clock orientations around the circumference of peripheral wall 210. Second notch 222 and third notch 224 may be 180 degrees from each other to increase fixation length of connecting platform 202 and/or flexibility of placement of connecting platform 202. First notch 220 may be offset 90 degrees from second notch 222 and third notch 224 to reduce likelihood of blocking the SMA.

As shown in Figure 3E, first notch 220 radially aligns between first inner wall 212 and second inner wall 214 to provide alignment of first branch extension 204 and second branch extension 206 with first CIA 18 and second CIA 20, respectively.

Connecting platform 202 includes proximal stent 226 connected to an outer surface of proximal peripheral wall region 211 and extending proximally beyond connecting platform 202. Distal crowns 228 and a portion of struts 230 may be secured to the outer surface of proximal peripheral wall region 211. These portions may be molded to proximal peripheral wall region 211 when connecting platform 202 is formed of a polymeric material. Distal crowns 228 may be stitched or sutured to proximal peripheral wall region 211 of connecting platform 202. Proximal stent 226 is configured to extend beyond the circumference of peripheral wall 210 to anchor to an inner wall of abdominal aorta 10.

As shown in Figures 3B through 3D, first sealing region 231 and second sealing region 232 extend radially from peripheral wall 210. First sealing region 231 and second sealing region 232 may be spaced apart from each other to from valley 234 therebetween. First sealing region 231 and second sealing region 232 may extend the entire circumference of peripheral wall 210 to enhance the sealing between the inner vessel wall and connecting platform 202. First sealing region 231 and second sealing region 232 may be capped with a polymeric material having a higher coefficient of friction than the peripheral wall 210 to enhance the anchoring of connecting platform 202 to the inner vessel wall. The polymeric material may include hooks or other surface features to enhance the grip of the peripheral wall of the connecting platform to the inner wall of the vessel. As shown in Figure 3D, first sealing region 231 and second sealing region 232 are longitudinally aligned with proximal wall 208 to reinforce proximal wall 208.

Peripheral wall 210 may include sealing height 236 of a sealing region configured to seal with an inner vessel wall at a landing zone between the renal arteries and the SMA. In one or more embodiments, the sealing height H may be any of the following values or in a range of any two of the following values: 20.0, 20.5, 21.0, 21.5, 22.0, 22.5, 23.0, 23.5, 24.0, 24.5, 25.0, 25.5, and 26.0 mm.

The connected platforms of one or more embodiments (e.g., connected platforms 26 and 202) may be provided in several different sizes (e.g., diameters) to be able to select a connecting platform that fits a specific anatomy of a patient. The branch extensions may also be available in alternative sizes to accommodate different diameters of the renal arteries. In one or more embodiments, the branch extensions may be available in 5 mm and 8 mm sizes whereas the connected platform may be available in five different diameters, thereby having 10 potential size combination to enable off the shelf use of the stent graft system. In other embodiments, there may be a range of distances between the proximal end of the branch extensions and the renal limbs to avoid the need to trim the extensions and provide more off the shelf options to customize the system.

The length of the main body of a first branch extension may be customized independent of the length of the main body of a second branch extension. The following method may be used to customize the length of a main body. A CT scan of a patient's anatomy may be used to measure the length of a stent graft to be trimmed. The branch extension may be unloaded from the delivery system. The proximal end of the branch extension may be cut according to the CT scan measurement, thereby shortening the length of the branch extension. The cut branch extension may be re-loaded into the delivery system for deployment. Alternatively, instead of trimming/cutting the branch extension, a wall of the excess proximal height of the branch extension may be fastened to the inner vessel wall with a suture, screw or other fastener to resist flapping around of the excess height.

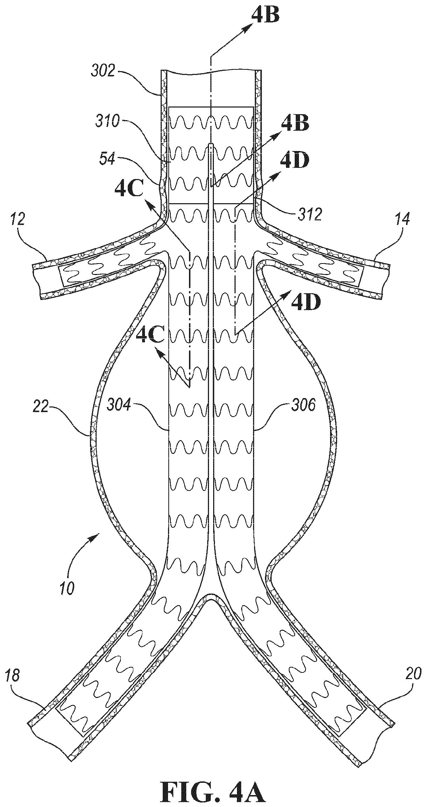

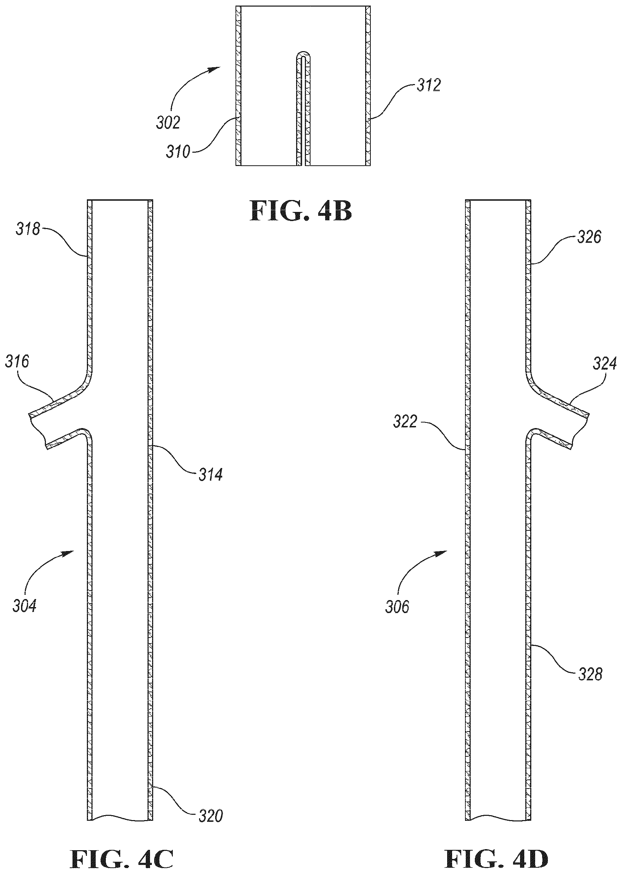

As shown in Figure 4A, stent graft system 300 bridges aneurysm 22 within abdominal aorta 10. As shown in Figures 4A through 4D, stent graft system 300 includes connecting platform 302, first branch extension 304, and second branch extension 306.

As a first step in one or more embodiments, a delivery system including connecting platform 302 in a compressed, delivery state may be introduced via femoral access. The delivery system may be advanced into abdominal aorta 10 over a guidewire. A delivery sheath of the delivery system may be withdrawn to release connecting platform 302 into an expanded, deployed position at landing zone 54 to resist blood flow between connecting platform 302 and the inner vessel wall. Connecting platform 302 includes main body 308 and first bifurcated leg 310 and second bifurcated leg 312 extending from main body 308.

First branch extension 304 includes first main body 314 and first renal limb 316. First main body 314 includes proximal end region 318 and distal end region 320. As a second step in one or more embodiments, first branch extension 304 is implanted with distal end region 320 within first CIA 18, proximal end region 318 coupled within first bifurcated leg 310 of connecting platform 302, and first renal limb 316 within or adjacent the right renal artery 12. In embodiments where the limb is a branch, port, coupling, fenestration, etc., a bridging stent graft may be extended into the renal artery.

Second branch extension 306 includes second main body 322 and second renal limb 324. Second main body 322 includes proximal end region 326 and distal end region 328. As a third step in one or more embodiments, second branch extension 306 is implanted with distal end region 328 in second CIA 20, proximal end region 326 coupled within second bifurcated leg 312 of connecting platform 302, and second renal limb 324 within or adjacent the left renal artery 14. In embodiments where the limb is a branch, port, coupling, fenestration, etc., a bridging stent graft may be extended into the renal artery.

Proximal end region 318 of first branch extension 304 may be implanted in connecting platform 302 independently of proximal end region 326 of second branch extension 306 such that the height of first branch extension 304 and second branch extension 306 within the patient's vasculature may vary independent of each other. For instance, one of the branch extensions may be deeper or less deep in a bifurcated leg of the connecting platform than the other branch extension. In another embodiment, a proximal portion of a branch extension may be unloaded and trimmed, as described above. In another embodiment, the distance between the proximal end of the branch extension and the renal limb may differ from the other without any trimming. This provides height adjustability to conform with different abdominal aorta anatomies of patients.

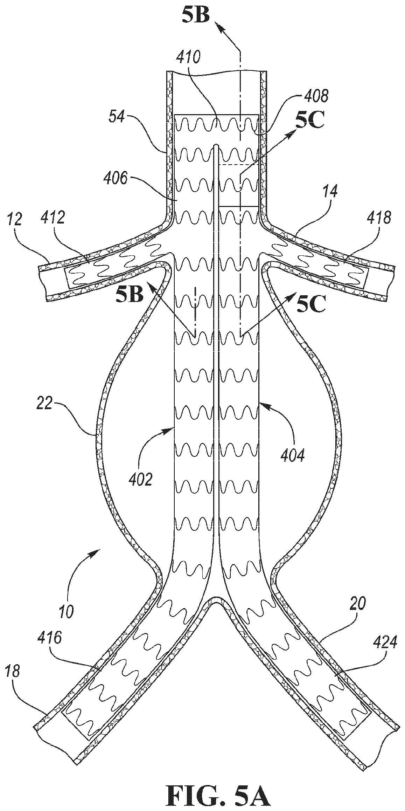

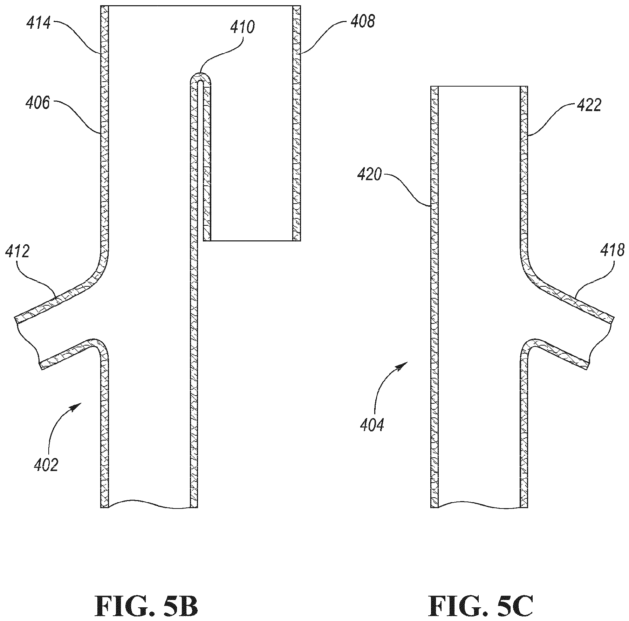

As shown in Figure 5A, stent graft system 400 bridges aneurysm 22 within abdominal aorta 10. As shown in Figures 5A through 5C, stent graft system 400 includes bifurcated first branch extension 402 and second branch extension 404 connected to bifurcated first branch extension 402. Bifurcated first branch extension 402 may include main bifurcated leg 406 and connecting bifurcated leg 408. Main bifurcated leg 406 and connecting bifurcated leg 408 may be connected at bridging portion 410. Main bifurcated leg 406 includes first renal limb 412, first proximal end region 414, and first distal end region 416. Second branch extension 404 includes second renal limb 418 and second main body 420 having second proximal end region 422 and second distal end region 424.

As a first step in one or more embodiments, bifurcated first branch extension 402 is deployed at landing zone 54 to resist blood flow and to create a seal between bifurcated first branch extension 402 and the inner vessel wall, and implant first renal limb 412 within or adjacent the right renal artery 12 and distal end region 416 within first CIA 18. In embodiments where the limb is a branch, port, coupling, fenestration, etc., a bridging stent graft may be extended into the renal artery.

As a second step in one or more embodiments, second branch extension 404 is implanted with second distal end region 424 in second CIA 20, second proximal end region coupled within connecting bifurcated leg 408 of bifurcated first branch extension 402, and second renal limb 418 within or adjacent the left renal artery 14. In embodiments where the limb is a branch, port, coupling, fenestration, etc., a bridging stent graft may be extended into the renal artery. Similar to embodiments described above, the second branch extension 404 may be trimmed to customize the length or an off-the-shelf length may be chosen such that the second renal limb 418 aligns with the renal artery.

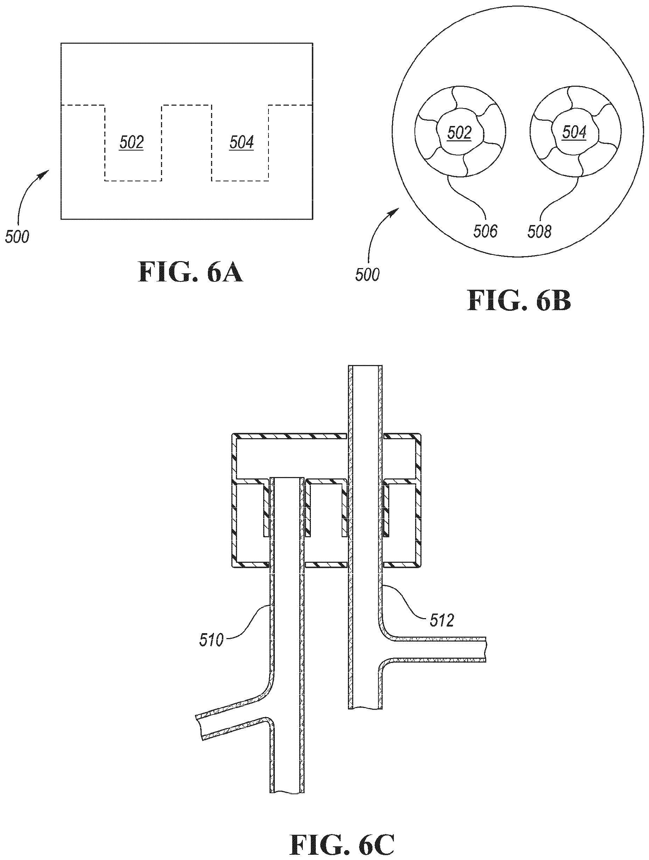

As shown in Figures 6A through 6C, connecting platform 500 defines first aperture 502 and second aperture 504. Connecting platform 500 may be formed of a stent graft material. First aperture 502 and second aperture 504 have first elastic structure 506 and second elastic structure 508, respectively. The elastic structures may be formed of elastic tubes. The elastic structures may be formed of a polymeric material. First branch extension 510 may be inserted within first elastic structure 506 such that first elastic structure 506 expands to accommodate first branch extension while maintaining a seal therebetween to resist blood leakage. Second branch extension 512 may be inserted within second elastic structure 508 such that second elastic structure 508 expands to accommodate second branch extension while maintaining a seal therebetween to resist blood leakage. As shown in Figure 6C, the height of first branch extension 510 and second branch extension 512 may be independently adjusted through first elastic structure 506 and second elastic structure 508. Similar to embodiments described above, the branch extensions may be trimmed to customize the length or an off-the-shelf length may be chosen such that the renal limbs align with the renal arteries.

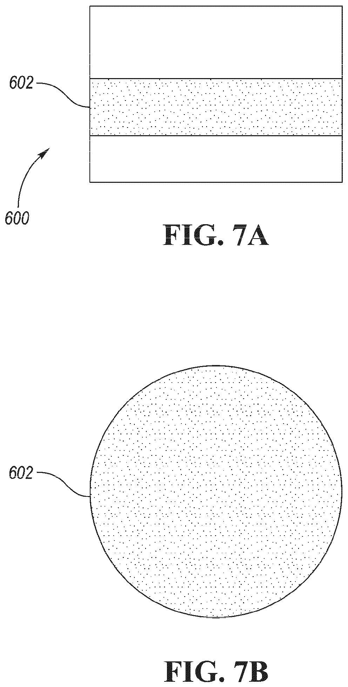

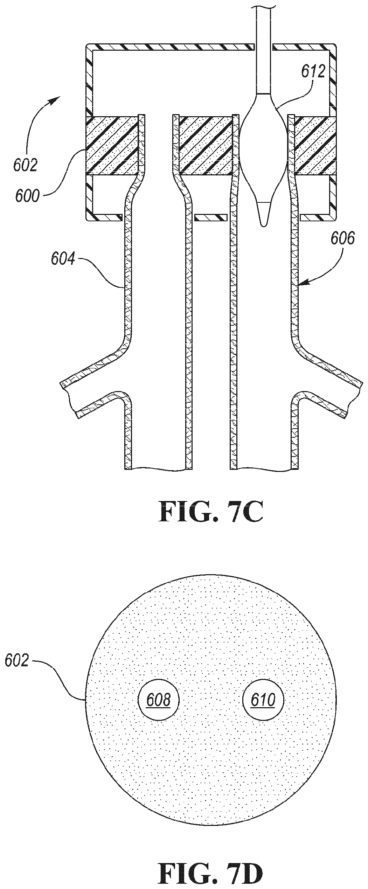

As shown in Figures 7A through 7D, connecting platform 600 includes foam insert 602. Connecting platform 600 may be formed of a stent graft material. Foam insert 602 may be configured to be punctured by a delivery system for first branch extension 604 and second branch extension 606 to form first aperture 608 and second aperture 610 within foam insert 602. Except for first aperture 608 and second aperture 610 occupied by first branch extension 604 and second branch extension 606, foam insert 602 may resist the flow of blood therethrough. After placement of first branch extension 604 and second branch extension 606, a balloon (e.g., balloon 612) may be utilized to fully open an inner lumen of one or both branch extensions.

In yet another embodiment, the stent graft system may include first and second branch extensions also configured to function as a connecting platform. For instance, the proximal ends of the first and second branch extensions may have opposing "D" shapes to combine to fill a circular cross-section of the aorta in an expanded, deployed state of the first and second branch extensions.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

The following examples are illustrative of the techniques described herein.

Example 1. A connecting platform for a stent graft system, the connecting platform comprising: a proximal wall having a circumference; a peripheral wall connected to the proximal wall at the circumference; first and second inner walls connected to the proximal wall, the first inner wall is configured to anchor a first branch extension of the stent graft system, and the second inner wall is configured to anchor a second branch extension of the stent graft system.

Example 2. The connecting platform of Example 1, wherein the first and second inner walls are spaced apart from each other and from the peripheral wall.

Example 3. The connecting platform of Example 1, wherein the peripheral wall includes inner and outer surfaces, and one or more annular sealing rings extend radially from the outer surface of the peripheral wall.

Example 4. The connecting platform of Example 1 further comprising a proximal stent connected to the proximal wall and/or the peripheral wall and extending proximally therefrom.

Example 5. The connecting platform of Example 1, wherein the first inner wall has a first inner wall diameter, the second inner wall has a second inner wall diameter, the first and second inner wall diameters are coextensive.

Example 6. The connecting platform of Example 5, wherein the proximal wall has a proximal wall diameter coextensive with the first inner wall diameter and the second inner wall diameter.

Example 7. The connecting platform of Example 5, wherein the proximal wall has a proximal wall diameter spaced apart from the first inner wall diameter and the second inner wall diameter.

Example 8. The connecting platform of Example 1, wherein the peripheral wall extends in a proximal direction to form a proximal peripheral wall region and extends in a distal direction to form a distal peripheral wall region.

Example 9. The connecting platform of Example 8, wherein the proximal peripheral wall region defines a notch configured to align with a superior mesenteric artery (SMA).

Example 10. The connecting platform of Example 8, wherein the distal peripheral wall region defines one or more notches configured to align with one or more renal arteries.

Example 11. An adjustable stent graft system comprising: a connecting platform; a first branch extension including a first main body and a first renal branch, the first main body including a first proximal end region and a first distal end region, the first renal branch is configured to extend into a first renal artery, the first proximal end region is configured to anchor to the connecting platform, and the first distal end region is configured to extend into a first common iliac artery (CIA); and a second branch extension including a second main body and a second renal branch, the second main body including a second proximal end region and a second distal end region, the second renal branch configured to extend into a second renal artery, the second proximal end region is configured to anchor to the connecting platform, and the second distal end region is configured to extend into a second CIA, the first and second branch extensions are independently adjustable relative to the connecting platform.

Example 12. The adjustable stent graft system of Example 11, wherein the connecting platform includes first and second inner walls configured to anchor the first and second proximal end regions, respectively, the first and second proximal end regions have first and second proximal end region outer diameters, the first and second inner walls have first and second inner wall diameters, and the first and second proximal end region outer diameters are greater than the first and second inner call diameters, respectively, to anchor the first and second branch extensions to the connecting platform.

Example 13. The adjustable stent graft system of Example 11, wherein the first and second proximal end regions include outwardly extending fixation features.

Example 14. The adjustable stent graft system of Example 11, wherein the first renal branch has a first nominal renal branch diameter and the second renal branch has a second nominal renal branch diameter different than the first nominal renal branch diameter.

Example 15. The adjustable stent graft system of Example 11, wherein the first branch extension has a first branch extension main body length and the second branch extension has a second branch extension main body length different than the first branch extension main body length.

Example 16. A branch extension of an adjustable stent graft system, the branch extension comprising: a main body including a proximal end region and a distal end region, the proximal end region is configured to anchor to a connecting platform of the adjustable stent graft system, the distal end region is configured to extend into a common iliac artery (CIA); and a renal branch extending from the main body and configured to extend to a renal artery, the renal branch having a renal branch distal end flared portion configured to anchor to the renal artery.

Example 17. The branch extension of Example 16, wherein the renal branch is formed of a flexible material configured to transition from a collapsed, delivery position into an extended, deployed position within the renal artery.

Example 18. The branch extension of Example 16, wherein the distal end region includes a distal end region flared portion configured to anchor to the CIA.

Example 19. The branch extension of Example 16, wherein the renal branch has a length of 20 to 25 mm.

Example 20. The branch extension of Example 16, wherein the renal branch is configured to independently maintain blood flow between an abdominal aorta and the renal artery without a bridging stent.

Further disclosed herein is the subject matter of the following clauses:
1. A connecting platform (26, 122, 202) for a stent graft system, the connecting platform comprising:
   a proximal wall (32, 208) having a circumference;
   a peripheral wall (34, 210) connected to the proximal wall at the circumference;
   first inner wall (36, 212) and second inner wall (38, 214) connected to the proximal wall, the first inner wall is configured to anchor a first branch extension (28, 124, 204) of the stent graft system, and the second inner wall is configured to anchor a second branch extension (30, 126, 206) of the stent graft system.
2. The connecting platform of clause 1, wherein the first and second inner walls are spaced apart from each other and from the peripheral wall.
3. The connecting platform of clause 1, wherein the peripheral wall includes inner and outer surfaces, and one or more annular sealing rings (56 and 58) extend radially from the outer surface of the peripheral wall.
4. The connecting platform of clause 1 further comprising a proximal stent connected to the proximal wall and/or the peripheral wall and extending proximally therefrom.
5. The connecting platform of clause 1, wherein the first inner wall has a first inner wall diameter, the second inner wall has a second inner wall diameter, the first and second inner wall diameters are coextensive.
6. The connecting platform of clause 5, wherein the proximal wall has a proximal wall diameter coextensive with the first inner wall diameter and the second inner wall diameter.
7. The connecting platform of clause 5, wherein the proximal wall has a proximal wall diameter spaced apart from the first inner wall diameter and the second inner wall diameter.
8. The connecting platform of clause 1, wherein the peripheral wall extends in a proximal direction to form a proximal peripheral wall region and extends in a distal direction to form a distal peripheral wall region.
9. The connecting platform of clause 8, wherein the proximal peripheral wall region defines a notch (220) configured to align with a superior mesenteric artery (SMA).
10. The connecting platform of clause 8, wherein the distal peripheral wall region defines one or more notches (222, 224) configured to align with one or more renal arteries.
11. An adjustable stent graft system comprising:
   the connecting platform of any of the preceding clauses;
   a first branch extension including a first main body (59, 128) and a first renal branch (61, 130), the first main body including a first proximal end region (60) and a first distal end region (62, 138), the first renal branch is configured to extend into a first renal artery (12 or 14), the first proximal end region is configured to anchor to the connecting platform, and the first distal end region is configured to extend into a first common iliac artery (CIA); and
   a second branch extension including a second main body (64, 146) and a second renal branch (66, 148), the second main body including a second proximal end region (68) and a second distal end region (70, 154), the second renal branch configured to extend into a second renal artery, the second proximal end region is configured to anchor to the connecting platform, and the second distal end region is configured to extend into a second CIA,
   the first and second branch extensions are independently adjustable relative to the connecting platform.
12. The adjustable stent graft system of clause 11, wherein the connecting platform includes first and second inner walls configured to anchor the first and second proximal end regions, respectively, the first and second proximal end regions have first and second proximal end region outer diameters, the first and second inner walls have first and second inner wall diameters, and the first and second proximal end region outer diameters are greater than the first and second inner call diameters, respectively, to anchor the first and second branch extensions to the connecting platform.
13. The adjustable stent graft system of clause 11, wherein the first and second proximal end regions include outwardly extending fixation features (44, 52).
14. The adjustable stent graft system of clause 11, wherein the first renal branch has a first nominal renal branch diameter and the second renal branch has a second nominal renal branch diameter different than the first nominal renal branch diameter.
15. The adjustable stent graft system of clause 11, wherein the first branch extension has a first branch extension main body length and the second branch extension has a second branch extension main body length different than the first branch extension main body length.

Further disclosed herein is an adjustable stent graft system. A first branch extension includes a first main body and a first renal branch. The first main body includes first proximal and distal end regions. The first renal branch is configured to extend into a first renal artery. The first proximal end region is configured to anchor to a connecting platform. The first distal end region is configured to extend into a first common iliac artery (CIA). A second branch extension includes a second main body and a second renal branch. The second main body includes second proximal and distal end regions. The second renal branch is configured to extend into a second renal artery. The second proximal end region is configured to anchor to the connecting platform. The second distal end region is configured to extend into a second CIA. The first and second branch extensions are independently adjustable relative to the connecting platform.

## Claims

1. A connecting platform (26, 122, 202) for a stent graft system, the connecting platform comprising:
a proximal wall (32, 208) having a circumference;
a peripheral wall (34, 210) connected to the proximal wall at the circumference;
first inner wall (36, 212) and second inner wall (38, 214) connected to the proximal wall, the first inner wall is configured to anchor a first branch extension (28, 124, 204) of the stent graft system, and the second inner wall is configured to anchor a second branch extension (30, 126, 206) of the stent graft system.

2. The connecting platform of claim 1, wherein the first and second inner walls are spaced apart from each other and from the peripheral wall.

3. The connecting platform of any preceding claim, wherein the peripheral wall includes inner and outer surfaces, and one or more annular sealing rings (56 and 58) extend radially from the outer surface of the peripheral wall.

4. The connecting platform of any preceding claim further comprising a proximal stent connected to the proximal wall and/or the peripheral wall and extending proximally therefrom.

5. The connecting platform of any preceding claim, wherein the first inner wall has a first inner wall diameter, the second inner wall has a second inner wall diameter, the first and second inner wall diameters are coextensive.

6. The connecting platform of claim 5, wherein the proximal wall has a proximal wall diameter coextensive with the first inner wall diameter and the second inner wall diameter.

7. The connecting platform of claim 5, wherein the proximal wall has a proximal wall diameter spaced apart from the first inner wall diameter and the second inner wall diameter.

8. The connecting platform of any preceding claim, wherein the peripheral wall extends in a proximal direction to form a proximal peripheral wall region and extends in a distal direction to form a distal peripheral wall region.

9. The connecting platform of claim 8, wherein the proximal peripheral wall region defines a notch (220) configured to align with a superior mesenteric artery (SMA).

10. The connecting platform of claim 8 or 9, wherein the distal peripheral wall region defines one or more notches (222, 224) configured to align with one or more renal arteries.

11. An adjustable stent graft system comprising:
the connecting platform of any of the preceding claims;
a first branch extension including a first main body (59, 128) and a first renal branch (61, 130), the first main body including a first proximal end region (60) and a first distal end region (62, 138), the first renal branch is configured to extend into a first renal artery (12 or 14), the first proximal end region is configured to anchor to the connecting platform, and the first distal end region is configured to extend into a first common iliac artery (CIA); and
a second branch extension including a second main body (64, 146) and a second renal branch (66, 148), the second main body including a second proximal end region (68) and a second distal end region (70, 154), the second renal branch configured to extend into a second renal artery, the second proximal end region is configured to anchor to the connecting platform, and the second distal end region is configured to extend into a second CIA,
the first and second branch extensions are independently adjustable relative to the connecting platform.

12. The adjustable stent graft system of claim 11, wherein the connecting platform includes first and second inner walls configured to anchor the first and second proximal end regions, respectively, the first and second proximal end regions have first and second proximal end region outer diameters, the first and second inner walls have first and second inner wall diameters, and the first and second proximal end region outer diameters are greater than the first and second inner call diameters, respectively, to anchor the first and second branch extensions to the connecting platform.

13. The adjustable stent graft system of claim 11 or 12, wherein the first and second proximal end regions include outwardly extending fixation features (44, 52).

14. The adjustable stent graft system of any one of claims 11 to 13, wherein the first renal branch has a first nominal renal branch diameter and the second renal branch has a second nominal renal branch diameter different than the first nominal renal branch diameter.

15. The adjustable stent graft system of any one of claims 11 to 14, wherein the first branch extension has a first branch extension main body length and the second branch extension has a second branch extension main body length different than the first branch extension main body length.
